# EUROPÄISCHE PATENTSCHRIFT

(11) **EP 2 362 529 B1**
(45) Veröffentlichungstag und Bekanntmachung des Hinweises auf die Patenterteilung: **08.01.2020**
(21) Anmeldenummer: 11155936.5
(22) Anmeldetag: 25.02.2011
(51) Int. Cl.: H02K 41/03, A61B 1/00, H02K 7/08, H02K 7/106, H02K 7/14, H02K 1/27

(54) **Reluktanz- und Lorentzkraftbetriebener Linearantrieb**
Linear drive powered by reluctance and Lorentz force
Entraînement linéaire entraîné par réluctance et force de Lorentz

(30) Priorität: 26.02.2010 DE 102010000583
(43) Veröffentlichungstag der Anmeldung: 31.08.2011
(73) Patentinhaber: Karl Storz SE & Co. KG, 78532 Tuttlingen (DE)
(72) Erfinder: Vogel, Walter, 78647 Trossingen (DE)
(74) Vertreter: Witte, Weller & Partner Patentanwälte mbB

(56) Entgegenhaltungen:
- EP-A2- 2 034 594
- DE-A1- 2 621 272
- DE-A1- 10 323 629
- DE-A1-102006 006 877

## Beschreibung

### Technisches Gebiet

Die Erfindung betrifft einen Linearmotor insbesondere für optische Systeme. Derartige optische Systeme werden beispielsweise in Endoskopen eingesetzt. Bei modernen Videoendoskopen sind ein Kamerachip sowie ein zugehöriges Linsensystem in die Endoskopspitze integriert. Zur Einstellung der Brennweite bzw. des Fokus des Linsensystems wird ein miniaturisierter Motor benötigt.

### Stand der Technik

Klassische Endoskope, wie sie beispielsweise für die minimalinvasive Chirurgie eingesetzt werden können, führen das Bild mittels Stablinsen vom intrakorporalen Objektiv zum extrakorporalen Okular. Durch die Stablinsen ist das System starr und in der optischen Qualität begrenzt. Moderne Videoendoskope verwenden einen Kamerachip in der Endoskopspitze. Ein solches Endoskop ist in der US 7,365,768 B1 offenbart. Dieses hat vor dem Kamerachip eine starr angeordnete Linse. Eine Einstellung der Brennweite der Linse ist nicht möglich.

Die DE 196 18 355 C2 zeigt einen in Endoskope integrierbaren Linearantrieb zum Einstellen der Brennweite eines Linsensystems. Dazu wird ein Permanentmagnet als Läufer innerhalb einer Statorspule bewegt. Der Linearantrieb ist wegen der großen Masse des Permanentmagneten jedoch träge. Der Zusammenhang zwischen dem Spulenstrom und der Läuferposition ist nicht eindeutig und macht einen zusätzlichen Wegsensor mit Lageregelung notwendig.

Die DE 37 17 872 C2 offenbart einen Antrieb mit einem Läufer und einem Stator für ein Linsensystem in Videokameras. Der Läufer besteht aus zwei Eisenhülsen, welche durch einen Träger zur Aufnahme des Linsensystems miteinander verbunden sind. Der Stator hat zwei Spulen sowie einen einzigen ringförmigen Permanentmagneten zur Erzeugung der für die Bewegung notwendigen Magnetfelder zwischen den Spulen. Der komplexe Aufbau des Antriebs kann bei Videokameras mit Linsendurchmessern im Zentimeterbereich gut realisiert werden, ist aber nicht auf die für endoskopische Anwendungen benötigte Größe im Millimeterbereich skalierbar.

In der DE 103 23 629 A1 ist ein Wanderfeld-Linearmotor offenbart, der mindestens drei Statorspulen aufweist. Durch eine phasenverschobene Bestromung der Spulen wird ein Wanderfeld erzeugt, welches eine Verschiebung des Läufers mit axialen Permanentmagneten bewirkt. Zur Erzeugung des Wanderfeldes wird eine aufwendige Ansteuerschaltung benötigt.

Aus der DE 10 2008 038 926 A1 ist ein Linearantrieb bekannt, der zwei axial polarisierte Permanentmagnete im Läufer aufweist. Der Läufer wird durch die Bestromung der Statorspulen in axialer. Richtung ausgelenkt. Zusätzlich werden die stabilen Positionen des Läufers durch die im Stator angebrachten Polschuhe realisiert, so dass eine kontinuierliche Verschiebung des Läufers in einem Hüllrohr ermöglicht wird. Nachteilig ist dabei die Abhängigkeit des Hubs und der Stellkräfte von den weichmagnetischen Stator-Polschuhen, wodurch eine hohe Genauigkeit bei der Fertigung und der Montage dieser Teile vorausgesetzt wird.

In der DE 10 2006 00 68 77 A1 ist eine magnetische Antriebsvorrichtung offenbart. Diese weist eine von einem Mantel umschlossene Spule, einen ferromagnetischen Leitring sowie vier Permanentmagnete auf.

### Darstellung der Erfindung

Der Erfindung liegt die Aufgabe zugrunde, einen Linearmotor mit derart kleinen Abmessungen zu schaffen, dass dieser in Endoskopen eingesetzt werden kann.

Weiterhin soll der Linearmotor eine definierte Nullpunktlage sowie eine reproduzierbare Auslenkung in Abhängigkeit vom Ansteuersignal aufweisen. Zudem soll der Linearmotor große Antriebskräfte bei kleiner Masse aufweisen und somit eine schnelle, kontinuierliche und exakte Positionierung des optischen Systems in einem möglichst großen Bereich ermöglichen. Dabei darf der Strahlengang durch das optische System beim Verschieben der Komponenten nicht blockiert werden. Die Verlustleistung des Linearmotors soll gering sein, so dass wenig Wärme in der Spitze eines Endoskops entsteht. Für eine einfache Fertigung und Montage soll der Antrieb aus möglichst wenigen und geometrisch einfachen Einzelbauteilen zusammengesetzt sein.

Diese Aufgabe ist durch einen Linearmotor nach Anspruch 1 gelöst. Weiterbildungen des Linearmotors sind in den abhängigen Ansprüchen angegeben.

Ein erfindungsgemäßer Linearmotor hat bevorzugt einen rotationssymmetrischen Läufer und/oder einen rotationssymmetrischen Stator. Bevorzugt ist der Linearmotor rotationssymmetrisch mit ringförmigen Rückschlußelement, Polschuhen, Permanentmagneten sowie einer ringförmigen Spule ausgeführt. Der Läufer und insbesondere die Permanentmagnete sowie der Polschuh sind bevorzugt hohlzylindrisch, d.h. sie haben die Form einer zylindrischen Hülse. Der Strahlengang eines optischen Systems kann dann durch die Hülse verlaufen. Insbesondere kann eine Linse oder ein anderes optisches Element in der Hülse sitzen. Somit kann durch eine Verschiebung der Hülse die Brennweite und/oder auch der Fokus des optischen Systems eingestellt werden.

Der Linearmotor ermöglicht zwischen zwei Endstellungen eine exakte Einstellung der Position des Läufers relativ zum Stator. Bei dem Linearmotor entspricht jedem Spulenstrom eine eindeutige Position des Läufers in Bezug zum Stator. Somit kann durch eine Einstellung des Spulenstromes der Läufer in dem Verfahrbereich kontinuierlich verschoben werden. Durch diese eindeutige Zuordnung zwischen dem Spulenstrom und der Läuferposition kann auf die nach dem Stand der Technik notwendige Wegmessung zur Positionsbestimmung des Läufers verzichtet werden. Die Einzelbauteile haben eine einfache Geometrie (Ringe, Hülsen) und sind deshalb einfach herstell- und montierbar.

Der Polschuh und das Rückschlußelement müssen immer ferromagnetische und/oder weichmagnetische Materialien umfassen.

Der Linearmotor ist bis zu einer Größe von wenigen Millimetern Außendurchmesser problemlos miniaturisierbar. Die Verfahrstrecke zwischen den beiden Endstellungen des Läufers liegt bei einem Motor mit wenigen Millimetern Außendurchmesser typischerweise bei etwa 1 bis 3 mm.

Die Spule kann wahlweise auf einen Spulenkörper oder auch ohne Spulenkörper gewickelt sein. Sie kann auch mehrteilig sein, d.h. aus mehreren Wicklungen bestehen.

Eine detaillierte Darstellung der Funktion des Motors findet sich in der Beschreibung der Figuren.

In einer weiteren vorteilhaften Ausgestaltung der Erfindung ist zwischen dem Stator und dem Läufer eine Gleitschicht. Diese kann insbesondere im Falle einer rotationssymmetrischen Anordnung als Gleithülse ausgeführt sein. Um die Magnetfelder möglichst wenig zu beeinflussen soll die Gleitschicht aus einem nicht magnetfeldführenden Material, insbesondere aus einem nicht- ferromagnetischen Material bestehen. Ihre Oberfläche umfasst bevorzugt ein Material mit niedrigem Reibungskoeffizienten, beispielsweise PTFE (Polytetrafluorethylen), Siliziumnitrid, Siliziumkarbid, poly-para-Xylylen Polymere oder DLC (diamond like carbon) wie beispielsweise in der US 5,478,650 offenbart.

Die Gleitschicht kann Unebenheiten auf der dem Läufer zugewandten Seite des Stators ausgleichen.

Der beschriebene Linearmotor kann in einer alternativen Ausführungsform mit einem ebenen, z.B. plattenförmig aufgebauten Stator und einem ebenfalls ebenen bzw. plattenförmigen Polschuh des Läufers realisiert werden. Alternativ können auch mehrere um einen Zylinder oder einen vieleckigen Körper angeordnete Linearmotoren vorgesehen sein. So ergibt sich beispielsweise bei einer gleichmäßigen Anordnung von Linearmotoren um einen Zylinder eine stabile Führung.

Ein erfindungsgemäßer Linearmotor kann auch aus Vollmaterial bestehen und an einem Ende einen Stößel zur Nanopositionierung von Instrumenten zur Nanopositionierung von Instrumenten aufweisen. Eine solche Vorrichtung ist bevorzugt in der Molekularbiologie, der Mikroelektronik oder auch der Neurochirurgie einsetzbar.

Besonders günstig ist es, wenn die Spule mit einem Gleichstrom mit überlagertem Wechselstrom kleiner Amplitude und einer Frequenz bis maximal 1kHz gespeist wird. Dadurch kann die Haft- und die Gleitreibung reduziert werden.

Eine Weiterbildung ist gerichtet auf ein Verfahren zum Betrieb eines Linearmotors, wobei der Linearmotors mit einem Gleichstrom und einem überlagertem Wechselstrom kleiner Amplitude und einer Frequenz bis maximal 1kHz gespeist wird. Dadurch kann die Haftreibung beziehungsweise Gleitreibung im inneren des Motors reduziert werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt schematisch einen Linearmotor entsprechend der Erfindung,
Figur 2 zeigt den Linearmotor aus Figur 1 mit einer Linse,
Figur 3 zeigt den Feldverlauf im Linearmotor mit stromloser Spule,
Figur 4 zeigt den Feldverlauf des Linearmotors mit stromdurchflossener Spule.
Figur 5 zeigt den Kraftverlauf des Linearmotors,
Figur 6 zeigt eine weitere Variante des Linearmotors,
Figur 7 zeigt den Feldverlauf im Linearmotor nach Fig. 6 mit stromloser Spule,
Figur 8 zeigt den Feldverlauf im Linearmotor nach Fig. 6 mit stromdurchflossener Spule,

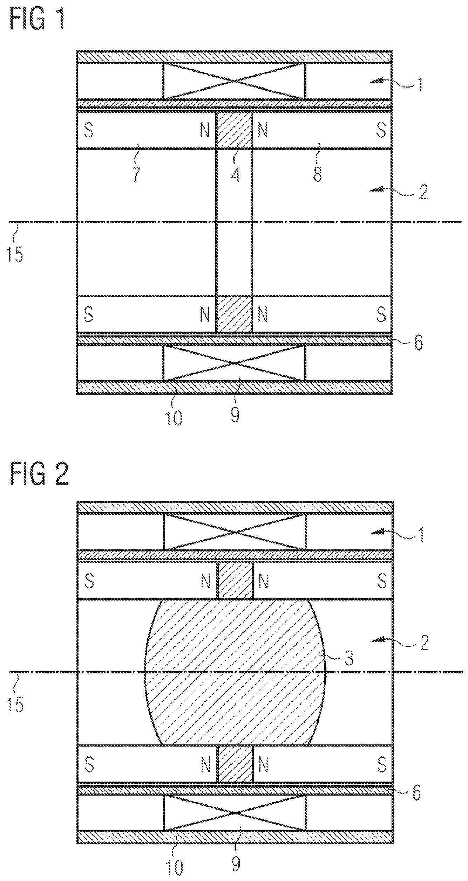

Figur 1 zeigt schematisch ein Ausführungsbeispiel einer erfindungsgemäßen Anordnung in zylindrischer Bauform im Schnitt. Der Stator 1 umfasst ein Rückschlußelement 10 in Form eines weichmagnetischen.Rückschlussrohrs, in dessen Bohrung eine Spule 9 angeordnet ist. Eine Gleithülse 6 schließt hier den Stator 1 nach innen ab und stellt eine Gleitschicht für den Läufer zur Verfügung. Die Gleithülse muss aus einem nicht- ferromagnetischen Material bestehen. Der Läufer 2 umfasst hier zwei entgegengesetzt magnetisierte Permanentmagnete 7, 8 und einen dazwischen liegenden Polschuh 4. In die Bohrung des Läufers kann ein zu positionierendes Element, wie eine optische Komponente, eingebracht werden. Der Läufer ist axial in beide Richtungen innerhalb der Gleithülse verschiebbar. Die Mittelachse 15 ist bei rotationssymmetrisch aufgebauten Anordnungen auch die Rotationsachse.

Figur 2 zeigt einen Linearmotor nach Figur 1, wobei noch eine Linse 3 dargestellt ist, die durch den Linearmotor in Richtung der Mittelachse 15 verschoben werden kann.

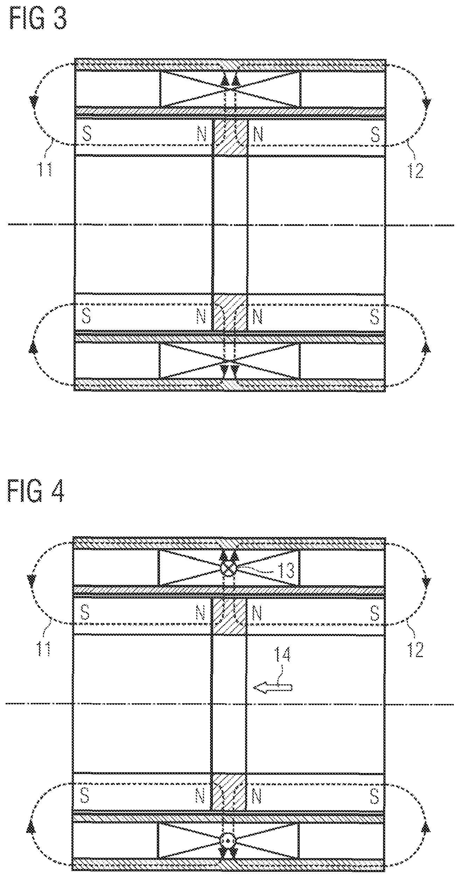

Figur 3 zeigt eine Darstellung der Erfindung mit den magnetischen Kreisen im Ruhezustand bei stromloser Spule.

Die Permanentmagnete erzeugen jeweils einen magnetischen Fluss 11, 12, der vom Nordpol eines jeden Permanentmagnets 7, 8 ausgehend durch den Polschuh 4 hindurch und jeweils radial nach außen durch die Spule 9 in das Rückschlußelement 10 des Stators fließt. Der magnetische Fluss geht weiter in Richtung der Außenseiten des Rückschlußelements 10 und von dort über den freien Luftraum zurück zum Südpol der Permanentmagnete 7, 8. Die beiden magnetischen Kreise 11, 12 sind bestrebt, die Länge ihres jeweiligen Luftspalts zu minimieren, um somit den jeweiligen magnetischen Widerstand des Kreises zu minimieren. Dabei treten in beiden magnetischen Kreisen sogenannte Reluktanzkräfte auf, die bestrebt sind, die Außenseiten der Permanentmagnete, in diesem Falle die Südpole, möglichst weit in das Innere des Rückschlusselementes 10 zu ziehen und damit den Luftspalt um so den magnetischen Widerstand zu reduzieren. In mittiger Stellung des Läufers sind die Luftspalte und damit die Reluktanzkräfte der beiden Kreise gleich groß. Bei geeigneter Auslegung der Länge des Läufers sowie der axialen Längen der Komponenten des Stators ergibt sich dann eine stabile zentrale Position des Läufers.

Wird der Läufer in Folge extern wirkender Kräfte, beispielsweise Beschleunigungskräfte, in eine seiner beiden Bewegungsrichtungen aus dieser stabilen Position heraus gedrängt, so wirken rücktreibende Kräfte, die den Läufer in die zentrale Position zurückdrängen. Die magnetischen Kräfte halten den Läufer vergleichbar zu einer mechanischen Feder in dieser Position.

Figur 4 zeigt die magnetischen Kreise bei bestromter Spule.

Wird nun die Spule 9 mit der in Abbildung 4 gekennzeichneten Richtung 13 bestromt, das heißt mit Strom durchflossen, so fließt der Strom in der oberen Hälfte der Querschnittsfläche in die Bildebene hinein und in der unteren aus ihr heraus. Durch die Durchsetzung der Spule 9 mit dem Magnetfeld aus den Permanentmagneten 7, 8 entsteht eine Lorentzkraft. Fließt ein Strom entsprechend der Zeichnung durch die Spule, so wird durch die Lorentzkraft der Läufer 2 nach in Richtung 4 nach links gezogen. Dieser Lorentzkraft entgegen wirken die Reluktanzkräfte, die den Läufer wieder zurück in die Mittellage zu ziehen versuchen. Es gibt nun für jeden Strom durch die Spule 9 eine Position des Läufers, bei der die Summe aus den positionsabhängigen Reluktanzkräften und der weitgehend positionsunabhängigen Lorentzkraft gleich null ist. Es stellt sich hier eine stabile Position ein. Somit besteht eine eindeutige Zuordnung zwischen Stromstärke und Läuferposition. Eine Umkehr der Stromrichtung bewirkt eine Verschiebung des Läufers 2 in die entgegengesetzte Richtung. Bei symmetrischem Aufbau sind die Bewegungseigenschaften in beide Richtungen identisch. Entscheidend für die Antriebseigenschaften ist weiterhin, dass die Position des Läufers auch in der der Stromstärke entsprechenden Sollposition reproduzierbar und stabil ist.

Die Polungsrichtung der Magnete lässt sich auch umkehren. Hierdurch kehrt sich die Bewegungsrichtung bei gegebenem Strom im Vergleich zum bisher beschriebenen Aufbau um. Entscheidend für die Funktionstüchtigkeit des Linearmotors ist lediglich, dass beide Magnete entgegengesetzt magnetisiert sind.

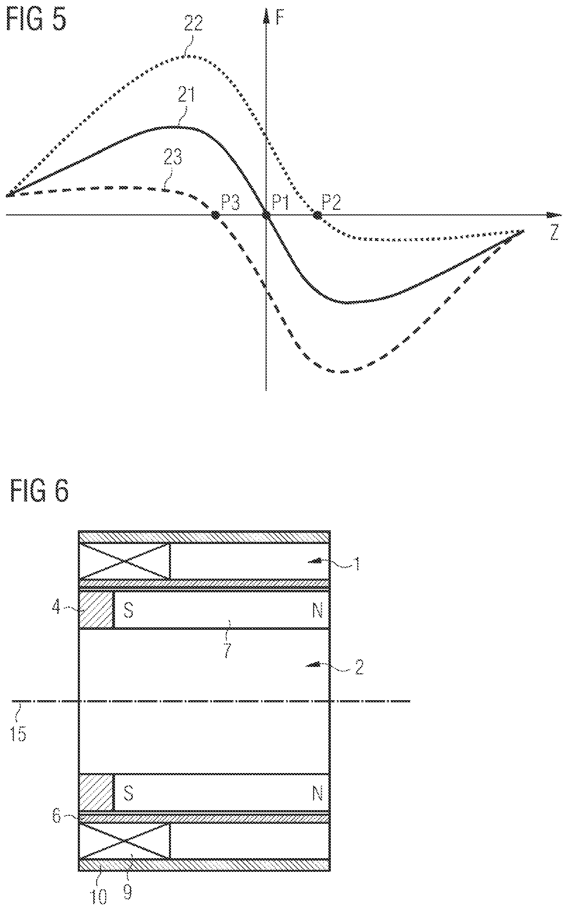

Figur 5 zeigt in einem Diagramm den Zusammenhang zwischen der Summe F der Kräfte aus Reluktanzkräften und Lorentzkraft sowie der Position z des Läufers. Eine positive Kraft F ist nach oben und eine positive Auslenkung z nach rechts aufgetragen. Ohne Bestromung nimmt der Läufer die Mittenposition P1 ein. Wird der Läufer nach links bewegt, so erhält dieser durch die Reluktanzkräfte eine positive Kraft in Richtung der z-Achse, nach rechts. Er wird also versuchen, gegen diese Kraft seine Mittenposition beizubehalten. Entsprechendes gilt für eine Auslenkung nach rechts. Dieser Kraftverlauf ist in der Kurve 21 dargestellt. Fließt nun ein Strom in der in Figur 4 dargestellten ersten Richtung durch die Spule, so ergibt sich, wie in der Beschreibung zur Figur 4 dargestellt, ein neues Gleichgewicht aus Reluktanzkräften und der Lorentzkraft im Punkt P3. Somit wird die ganze Kurve verschoben. Durch das zusätzliche Magnetfeld der Spule werden auch die Reluktanzkräfte verringert. Entsprechend sind die Rückstellkräfte im linken Teil der Kurve 23 geringer als in der Kurve 21 ohne Bestromung. In dem Teil der Kurve 23 rechts von dem Punkt P3 wirken Reluktanzkräfte und Lorentzkraft gleichsinnig. Entsprechend erhöht sich die Rückstellkraft. Wird nun ein Strom in entgegengesetzter, zweiter Richtung durch die Spule geleitet, so ergibt sich ein neues Gleichgewicht aus Reluktanzkräften und Lorentzkraft im Punkt P2, bei einer Position des Läufers rechts von der Nullposition. Die Kurve 22 des Kraftverlaufs ist entsprechend umgekehrt wie die Kurve 23.

Figur 6 zeigt eine weitere Variante der Erfindung. Diese ist ähnlich wie die in Figur 1 dargestellte Variante aufgebaut. Allerdings weist der Läufer hier nur einen ersten Permanentmagneten 7 auf. Es kann hier durch Bestromung der Läufer nur nach rechts bewegt werden.

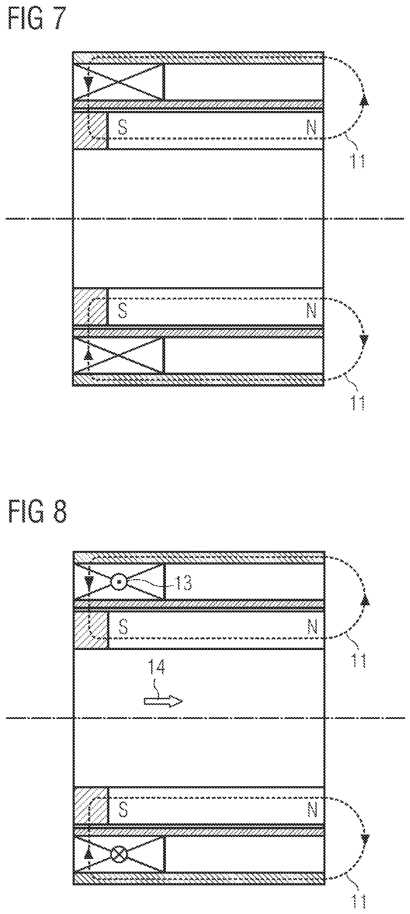

Figur 7 zeigt den Verlauf des Magnetfeldes bei unbestromter Spule. Der magnetische Fluss verläuft hier im oberen Teil der Figur entgegen dem Uhrzeigersinn. Es stellt sich eine Mittellage ein, bei der die Reluktanzkräfte an beiden Seiten des Läufers gleich groß sind. Aufgrund der unsymmetrischen Anordnung mit dem Polschuh an der linken Seite würde der Läufer geringfügig nach links aus dem Stator hinausragen. Dies kann er durch einen hier nicht dargestellten Anschlag verhindert werden.

Figur 8 zeigt den Verlauf des magnetischen Feldes bei stromdurchflossener Spule in Richtung 13. Der Strom fließt hier im unteren Teil der Spule in die Zeichnungsebene hinein und tritt aus dem oberen Teil der Spule aus der Zeichnungsebene heraus. Hier ergibt sich eine Lorentzkraft durch die Wechselwirkung des durch den Polschuh 4 durch die Spule 9 geleiteten Magnetfeldes mit dem Spulenstrom. Diese Lorentzkraft wirkt wieder den Reluktanzkräften entgegen, so dass sich auch hier ein spulenstromabhängiger Punkt mit Kräftegleichgewicht ergibt.

### Bezugszeichenliste

- 1: Stator
- 2: Läufer
- 4: Polschuh
- 6: Gleitschicht
- 7: Erster Permanentmagnet
- 8: Zweiter Permanentmagnet
- 9: Spule
- 10: Rückschlußelement
- 11: Erster magnetischer Kreis
- 12: Zweiter magnetischer Kreis
- 13: Stromrichtung
- 14: Bewegungsrichtung
- 15: Mittelachse
- 21: Kraft-Weg-Kennlinie stromlos
- 22: Kraft-Weg-Kennlinie bei Strom in einer ersten Richtung
- 23: Kraft-Weg-Kennlinie bei Strom in einer zweiten Richtung
- F: Kraft auf den Läufer
- z: Auslenkung des Läufers
- P1: Position des Läufers im stromlosen Zustand
- P2: Position des Läufers mit Strom in einer ersten Richtung
- P3: Position des Läufers mit Strom in einer zweiten Richtung

## Patentansprüche

1. Linearmotor aufweisend einen Stator (1) mit genau einer Spule (9), welche in ihrer ganzen Länge von einem Rückschlusselement (10) umschlossen ist, und einem von der Spule umfassten, parallel zum Stator verschiebbaren Läufer (2), mit einem Permanentmagneten (7) sowie einem Polschuh (4) aus weichmagnetischem Material an einer Seite des Permanentmagneten (7), wobei die Länge des Läufers (2) in etwa der Länge des Rückschlusselementes des Stators entspricht, wobei ein magnetischer Fluss (11) ausgehend von dem Permanentmagneten (7) über den Polschuh (4) radial nach außen durch die Spule (9) und das Rückschlusselement (10) zu einer Außenseite an einem axialen Ende des Rückschlusselements (10) besteht, der von der Außenseite über einen freien Luftraum zum Permanentmagneten (7) zurückgeführt ist, oder, bei umgekehrter Polungsrichtung des Permanentmagneten (7), in umgekehrter Flussrichtung, und wobei die Länge des Rückschlusselements (10) gleich der Länge des Läufers (2) oder zwischen 0 Prozent und 10 Prozent größer als die Länge des Läufers (2) ist.

2. Linearmotor nach Anspruch 1, wobei im Inneren des Läufers (2) ein optisches Element (3) aufgenommen werden kann.

3. Linearmotor nach einem der vorhergehenden Ansprüche, wobei der Läufer (2) zusätzlich zu dem Permanentmagneten (7) einen weiteren, gegensinnig gepolten Permanentmagneten (8) aufweist und der Polschuh (4) zwischen den beiden Permanentmagneten angeordnet ist.

4. Linearmotor nach einem der vorhergehenden Ansprüche, wobei der Läufer (2) rotationssymmetrisch ist.

5. Linearmotor nach einem der vorhergehenden Ansprüche, wobei der Stator (1) rotationssymmetrisch ist.

6. Linearmotor nach einem der vorhergehenden Ansprüche, wobei eine Gleithülse (6) mit einem Material mit niedrigem Reibungskoeffizienten an der Oberfläche zwischen Stator (1) und Läufer (2) angeordnet ist.

7. Linearmotor nach einem der vorhergehenden Ansprüche, wobei der Läufer (2) aus Vollmaterial besteht und lediglich einen Stößel zur Nanopositionierung von Instrumenten aufweist.

8. Linearmotor nach einem der vorhergehenden Ansprüche, wobei eine Speisung des Linearmotors zur Minderung der Haft- und Gleitreibung dafür ausgebildet ist, den Gleichstrom durch die Spule (9) mit einem sehr kleinen Wechselstrom mit Frequenzen bis maximal 1 kHz zu überlagern.

## Claims

1. Linear motor having a stator (1) with exactly one coil (9) which is enclosed in its entire length by a feedback element (10), and a rotor (2) which is surrounded by the coil and can be displaced parallel to the stator, with a permanent magnet (7) and a pole shoe (4) made of soft magnetic material on one side of the permanent magnet (7), the length of the rotor (2) corresponding approximately to the length of the feedback element of the stator, wherein there is a magnetic flux (11) starting from the permanent magnet (7) via the pole shoe (4) radially outwards through the coil (9) and the feedback element (10) to an outside at an axial end of the feedback element (10), which is returned from the outside via a free air space to the permanent magnet (7), or, with the polarity direction of the permanent magnet (7) reversed, in the reverse flux direction, and wherein the length of the return element (10) is equal to the length of the rotor (2) or between 0 percent and 10 percent greater than the length of the rotor (2).

2. Linear motor according to claim 1, in which an optical element (3) can be accommodated inside the rotor (2).

3. Linear motor according to one of the preceding claims, the rotor (2) having, in addition to the permanent magnet (7), a further permanent magnet (8) with opposite polarity, and the pole shoe (4) being arranged between the two permanent magnets.

4. Linear motor according to one of the preceding claims, the rotor (2) being rotationally symmetrical.

5. Linear motor according to one of the preceding claims, the stator (1) being rotationally symmetrical.

6. Linear motor according to one of the preceding claims, wherein a sliding sleeve (6) having a low coefficient of friction material is disposed at the surface between stator (1) and rotor (2).

7. Linear motor according to one of the preceding claims, the rotor (2) being made of solid material and having only one plunger for nanopositioning of instruments.

8. Linear motor according to one of the preceding claims, wherein a supply of the linear motor for reducing static and sliding friction is adapted to superimpose the direct current through the coil (9) with a very small alternating current having frequencies up to a maximum of 1 kHz.

## Revendications

1. Moteur linéaire, comprenant un stator (1) ayant exactement une bobine (9) qui est entourée sur toute sa longueur par un élément de retour de flux (10), et un rotor (2) englobé par la bobine et pouvant coulisser parallèlement au stator, comprenant un aimant permanent (7) ainsi qu'une pièce polaire (4) en matériau magnétique doux d'un côté de l'aimant permanent (7), la longueur du rotor (2) correspondant approximativement à la longueur de l'élément de retour de flux du stator, un flux magnétique (11) émanant de l'aimant permanent (7) par la pièce polaire (4) dans le sens radial vers l'extérieur à travers la bobine (9) et l'élément de retour de flux (10) vers un côté extérieur au niveau d'une extrémité axiale de l'élément de retour de flux (10) s'établissant, lequel est renvoyé vers l'aimant permanent (7) depuis le côté extérieur par le biais d'un espace d'air libre ou, lors d'un sens de polarisation inversé de l'aiment permanent (7), dans une direction de flux inverse, et la longueur de l'élément de retour de flux (10) étant égale à la longueur du rotor (2) ou étant entre 0 pour cent et 10 pour cent supérieure à la longueur du rotor (2).

2. Moteur linéaire selon la revendication 1, un élément optique (3) pouvant être accueilli à l'intérieur du rotor (2).

3. Moteur linéaire selon l'une des revendications précédentes, le rotor (2), en plus de l'aimant permanent (7), possédant un aimant permanent (8) supplémentaire polarisé en sens inverse et la pièce polaire (4) étant disposée entre les deux aimants permanents.

4. Moteur linéaire selon l'une des revendications précédentes, le rotor (2) étant symétrique en rotation.

5. Moteur linéaire selon l'une des revendications précédentes, le stator (1) étant symétrique en rotation.

6. Moteur linéaire selon l'une des revendications précédentes, une douille de glissement (6) avec un matériau à faible coefficient de friction étant disposée sur la surface entre le stator (1) et le rotor (2).

7. Moteur linéaire selon l'une des revendications précédentes, le rotor (2) étant constitué de matériau plein et possédant uniquement un taquet pour le positionnement nanométrique d'instruments.

8. Moteur linéaire selon l'une des revendications précédentes, une alimentation du moteur linéaire, en vue de réduire au minimum le frottement par adhérence et la friction de glissement, étant configurée pour superposer au courant continu à travers la bobine (9) un courant alternatif très faible dont les fréquences sont au maximum de 1 kHz.
